# EUROPÄISCHE PATENTSCHRIFT

(11) **EP 3 393 415 B1**
(45) Veröffentlichungstag und Bekanntmachung des Hinweises auf die Patenterteilung: **07.06.2023**
(21) Anmeldenummer: 16825781.4
(22) Anmeldetag: 23.12.2016
(51) Int. Cl.: A61F 13/02, A61F 7/10, A61L 15/12, A61F 13/00

(54) **BANDAGE**
BANDAGE
BANDAGE

(30) Priorität: 23.12.2015 DE 102015226645
(43) Veröffentlichungstag der Anmeldung: 31.10.2018
(73) Patentinhaber: KOB GmbH, 67752 Wolfstein (DE)
(72) Erfinder: LANGEN, Guenter, 67752 Wolfstein (DE)
(74) Vertreter: DREISS Patentanwälte PartG mbB
(86) Internationale Anmeldenummer: PCT/EP2016/082627
(87) Internationale Veröffentlichungsnummer: WO 2017/109210

(56) Entgegenhaltungen:
- DE-A1- 2 912 129
- GB-A- 1 320 628
- GB-A- 1 342 115
- GB-A- 2 010 933
- Anonymous: "Sporty Cool", , 2. April 2015 (2015-04-02), XP055355793, Gefunden im Internet: URL:https://www.amazon.de/Sporty-Cool-Set- 8cm-gedehnt/dp/B00VK6ZLWA [gefunden am 2017-03-16]

## Beschreibung

Die Erfindung betrifft eine Bandage umfassend ein Flachbahnmaterial als Substrat, wobei auf beiden Flachseiten des Substrats eine selbsthaftende kohäsive Klebemasse zumindest partiell aufgebracht ist und das Substrat mit einer flüssigen Zubereitung getränkt ist. Im Stand der Technik sind seit vielen Jahren kohäsive Haftbinden mit flüssigen Zubereitungen z. B. als Kühlbinden bekannt. Dazu wird eine trockene Binde mit einem Kühlfluid beispielsweise auf Basis Wasser/Ethanol oder Wasser/Isopropanol imprägniert, wobei diesen Mischungen gegebenenfalls weitere Hilfsstoffe zugesetzt sind. Derartige Produkte sind beispielsweise unter den Handelsnamen "Sporty Cool" der Karl Otto Braun GmbH & Co. KG, Wolfstein, Deutschland sowie unter "Ideal Cool" der Firma Paul Hartmann AG, Heidenheim, Deutschland bekannt. Diese Produkte verwenden ein elastisches Textilmaterial als Substrat, welches mit Naturkautschuklatex in Form der wässrigen Dispersion als kohäsives Haftklebemittel beschichtet ist. Die Binde wird dann im getrockneten Zustand mit der Kühlflüssigkeit imprägniert. Als Hilfsstoffe können beispielsweise ätherische Öle eingesetzt werden.

Derartige Binden werden als Kompressions- und/oder Stützbinden zur Verwendung bei stumpfen Verletzungen u. a. in der Sportmedizin verwendet. Durch die Kälte und die Kompression wird Schwellungen entgegengewirkt.

Darüber hinaus ist es beispielsweise aus der DE 295 18 881 U1 bekannt, eine Kompressionsbandage vorzusehen, die sowohl haftend als auch kühlend ausgebildet ist. Dabei soll es sich um eine Bandage handeln, die ohne chemisch ablaufende Prozesse und ohne Vorkühlung im Kühl- oder Gefrierschrank einen Körperteil kühlt. Die Binde weist hierzu ein Gel auf Silikonbasis auf und ist darüber hinaus adhäsiv ausgebildet. Zur Bereitstellung der Kühlwirkung muss sie alle vier bis sechs Stunden in Wasser getaucht werden, um die Kühlwirkung beizubehalten.

Des Weiteren ist in der WO 2005/007050 A1 eine Kühlbinde auf Basis eines elastischen Trägermaterials mit einer kühlenden flüssigen Zubereitung auf Basis Ethanol, Wasser, Methanol und Isoamylazetat beschrieben.

Nachteilig bei den vorgenannten Binden, die auf Basis Naturkautschuklatex bezüglich ihrer kohäsiven Haftkräfte gebildet sind, ist, dass während der Lagerzeit die kohäsiven Kräfte nachlassen, so dass eine sichere Lagenhaftung der nassen Binde über eine Tragezeit von bis zu mehreren Tagen, wie sie wünschenswert ist, nicht mehr sicher realisiert werden kann.

Weitere kohäsive Binden, die jedoch nicht mit einer Kühlflüssigkeit beschrieben sind, sind aus der DE 29 12 129 A1 sowie der GB 2,010,933 A vorbekannt.

Es soll daher Aufgabe der Erfindung sein, eine kohäsive selbsthaftende Kühlbinde bereitzustellen, die alterungsstabil ist, so dass ihre volle Funktionalität hinsichtlich der Hafteigenschaften, aber auch der Kühlwirkung, auch bei mehrmonatiger Lagerzeit erhalten bleibt.

Die Erfindung löst daher die Aufgabe durch eine Bandage mit den Merkmalen des Anspruchs 1 sowie einem Verfahren mit den Merkmalen des Anspruchs 10 wobei die Bandage so ausgebildet ist, dass die Klebemasse einen wasserfreien kohäsiven Haftstoff umfassend Natur- und/oder Synthesekautschuk umfasst, bei dem der Natur- und/oder Synthesekautschuk zum Aufbringen in organischem Lösungsmittel gelöst oder der Haftklebstoff zum Aufbringen schmelzflüssig ist.

Auf diese Weise kann ein Kautschukmaterial für die kohäsiven Eigenschaften eingesetzt werden, ohne auf ein Latexmaterial zurückgreifen zu müssen.

Dabei ist der Einsatz eines kohäsiven Haftklebstoffs auf Naturkautschuk- oder Synthesekautschukbasis, wobei Synthesekautschuk bevorzugt ist, in der Lage, eine kohäsive Beschichtungsmasse für eine Binde bereitzustellen, die in überraschender Weise ein anderes Alterungsverhalten als Naturkautschuklatex zeigt und somit auch nach längerer Lagerung noch eine zufriedenstellende Haftkraft bereitstellt, auch bei feuchten Binden. Die Haftkraft zwischen den Lagen einer erfindungsgemäßen trockenen Binde entspricht nahezu identisch der Haftkraft von Binden auf Basis Naturkautschuklatex. Werden nun sowohl die erfindungsgemäße Binde als auch die Binde nach dem Stand der Technik mit einer flüssigen Zubereitung, z. B. aus 50% Ethanol in Wasser imprägniert, haften überraschenderweise die Lagen der erfindungsgemäßen Binde nach wie vor aufeinander und dies auch noch nach mehrmonatiger Lagerzeit der imprägnierten Binde, wohingegen die Binde gemäß dem Stand der Technik nicht oder nur minimal haftet. Die erfindungsgemäße Binde hat daher den Vorteil, dass eine sichere Lagenhaftung der nassen Binde ohne Verwendung von Verbandsklammern über eine Tragezeit von bis zu mehreren Tagen gegeben ist, auch wenn die Binde bereits seit mehreren Monaten gelagert wurde.

Es kann dabei erfindungsgemäß vorgesehen sein, dass der Naturkautschuk (Polyisopren) oder vorzugsweise der Synthesekautschuk (Polyisopren, -budadien, -chloropren) als offenporige Beschichtung vorzugsweise lediglich partiell, in Form von Fragmenten oder Mustern ausgeführt ist.

Ein entsprechender Auftrag kann dabei in verschiedener Weise erfolgen, nämlich beispielsweise a) in Form eines Sprühauftrags von Lösungen des Festkautschuks in organischen Lösungsmitteln, wobei hier C₅- bis C₁₂-Kohlenwasserstoffe, Ether, Esther oder Ketone eingesetzt werden können und darüber hinaus b) einem Sprühauftrag von Schmelzklebstoffen.

Darüber hinaus können entsprechende Lösungen des Kautschukmaterials in organischen Lösungsmitteln auch im Rahmen eines Schablonendrucks verarbeitet werden, wobei alternativ auch die Schmelzklebstoffe im Schablonendruckverfahren aufgebracht werden können. Darüber hinaus ist sowohl für die Lösungen als auch für die Schmelzklebstoffe auch ein Rasterwalzenauftrag denkbar.

Weitere Auftragsverfahren sind Messer- oder Walzenrakeln der Kautschuklösungen sowie Gießverfahren (Curtain coating) der Kautschuklösungen möglich.

Die vorgenannten Verfahren sollen dabei derart ausgeführt werden, dass nach der Beschichtung, Trocknung und Abkühlung ein mikroporöser, offenporiger Auftrag von Kautschukfragmenten in einer Auftragsmenge je Oberfläche von 2 bis 20 g/m² entsteht, wobei die Kautschukfragmente bevorzugt an der Oberfläche angeordnet sind und haftend mit dem Substrat verbunden sein können, und zwar hier insbesondere über eine Anhaftung an den Fasern.

Durch die kohäsive Haftklebemasse wird ein rutschfester Sitz bei Wicklung am Körperteil erreicht.

Der Anteil des Kautschuks, sofern dieser mit organischen Lösemitteln in Lösung gebracht wird, beträgt 5 bis 20% Feststoffgehalt. Die aufgebrachte Kautschuklösung wird dann in einem Trockenschritt so behandelt, dass das Lösungsmittel verdunstet, so dass dann nach dem Trockenprozess auf der Substratoberfläche ein geschlossener oder offenporiger Kautschukfilm verbleibt. Dieser wird beim Verpressen mit sich selbst siegeln bzw. haftet aufeinander, so dass hierdurch die kohäsive Haftkraft bereitgestellt wird.

Die Binde kann dabei in Längs- und/oder Querrichtung elastisch sein. Dabei kann insbesondere vorgesehen sein, dass das Substrat ein Gewebe, Gewirke, Gestrick oder Vlies ist. Die Elastizität kann dabei durch die Einarbeitung von elastischen Fäden, wie beispielsweise hochgedrehten Baumwollfäden, als Spinn- oder Zwirnkreppfäden, durch texturierte Polyamid- oder Polyestergarne sowie Gummi- oder Polyurethanfäden bzw. Kombinationen hiervon hervorgerufen werden. So kann z.B. die Elastizität durch Übernähen eines starren Vliessstoffes mit dauerelastischen Elastanfäden in Längsrichtung unter Anwendung der Nähwirktechnik MALIWATT erhalten wurde. Die Nähwirktechnik MALIWATT ist in Malimo Nähwirktechnologie, Ploch, Böttcher, Scharch, VEB Fachbuchverlag Leipzig, 1978, 1. Auflage beschrieben.

Die erfindungsgemäße flüssige Zubereitung besteht aus einer ein- oder mehrphasigen Zubereitung auf Basis von Wasser mit Zusätzen von anorganischen Stoffen, wie beispielsweise Salzen, oder organischen Stoffen, nämlich beispielsweise Alkoholen, Ester, Ether, Ketone oder Terpene. Als Zubereitungen können jedoch auch Gele oder Schäume eingesetzt werden. Hierdurch werden definierte physikalische und physiologische Wirkungen auf den Körper ausgeübt, wie beispielsweise Kühlen, Wärmen, Befeuchten, Hydratisieren, Entfetten, Rückfetten, Schmerzlinderung oder Reizhemmung, wie beispielsweise Hemmung von Juckreiz.

Die für die beabsichtigte Wirkung notwendige Menge des Fluids wird auf die kohäsive Binde nach Aufbringen der Klebemasse durch Sprühen, Tauchen, Fouladieren, Pflatschen und/oder Gießen aufgebracht. Die Zubereitung kann partiell oder über die gesamte Bandage verteilt sein.

Zur Vermeidung von Trocknungsverlusten durch Verdunsten bzw. Wirkstoffverlusten kann die Binde danach in eine hermetisch dicht siegelnde Verpackung, wie beispielsweise Folienbeutel, Peelpackungen, Tiefziehpackungen, Dosen, Flaschen, etc. eingebracht werden und so eine Lagerzeit von bis zu 3 Jahren erreicht werden.

Eine so gefertigte Bandage, die alternativ auch als Binde bezeichnet sein kann, besitzt eine kohäsive Selbsthaftung sowohl im nassen als auch im getrockneten Zustand, die auch nach mehrjährigem Lagerungsprozess noch gegeben ist.

Die Erfindung soll im Folgenden anhand von Beispielen näher erläutert werden.

### Beispiel 1 : Kohäsive Vliesbinde mit Kühlfluid

Als bahnförmiges Substrat wird eine elastische Vlieskonstruktion Typ 752, Handelsname NOWOPRESS 752, Hersteller Karl Otto Braun GmbH & Co., Wolfstein, Deutschland, verwendet, wobei das Grundtextil durch Übernähen eines starren Polypropylenvliesstoffs mit dauerelastischen Elastanfäden in Längsrichtung unter Anwendung der Nähwirktechnik MALIWATT erhalten wurde. Einzelheiten dieses Trägertextils siehe nachstehende Tabelle:

| | |
|---|---|
| Materialaufbau elast. | 84% Polypropylen, 16% Elastan |
| Nonwoven Typ 752 | |
| Grund-Vlies | PP-Spunbondvlies, 35 g/m², thermisch geprägt, Farbe blau (Pantone 18-1662 TPX) |
| Nähfaden | 133 dtex Elastan (Dorlastan, Hersteller ASAHI Kasai, Dormagen, Deutschland) |
| Nähfadendichte | 45 Fäden je 10 cm Breite |
| Nähfaden-Stichlänge, Bindung | 3 mm, offene Franse |
| | |
| Elastizität | Längsrichtung (Kettrichtung) |
| Dehnbarkeit nach DIN61632 bei K=3N/cm | 220 % |

Dieses elastische Grundtextil wird als Bahnenware der Breite 10 cm auf beiden Oberflächen unter Verwendung eines Walzenrakels mit einem kohäsiven Haftklebstoff auf Basis Polyisopren-Kautschuk beschichtet, um den gewünschten kohäsiven Hafteffekt zu erreichen. Der kohäsive Haftklebstoff besteht aus folgenden Komponenten:
1. Festkautschuk cis 1.4- Polyisopren , Handelsname NATSYN 2200, Hersteller: GOODYEAR, Ohio, USA
2. Lösemittel Isohexan , Handelsname ISOHEXAN , C6 Kohlenwasserstoffgemisch verschiedener Isomere, Hersteller: BIESTERFELD,Hamburg, Deutschland
3. Alterungsschutzmittel: 2,6 Ditertbutylpkresol, Handelsname IONOL CP, Hersteller: OXIRIS Chemicals, Sant Celoni, Spanien

Die Herstellung erfolgt gemäß folgender Vorschrift: In einem Edelstahlrührbehälter werden 400 g Isohexan (Komponente 2) vorgelegt und unter Rühren insgesamt 60,6 g Festkautschuk (Komponente 1) in Form von Granulat der Korngröße ca. 4 mm zugesetzt. Ebenfalls werden 1,21 g Alterungsschutzmittel (Komponente 3) als Pulver zugegeben. Bei einer Rührgeschwindigkeit von 300 Touren/min löst sich Komponente 3 sofort im Isohexan, während zur vollständigen Lösung der Komponente 2 ca. 120 Minuten benötigt werden. Im Ergebnis liegt eine gräulich trübe viskose Klebstoffmasse mit einem Feststoffgehalt von 13,1 % vor. Die Viskosität wurde mit einem Haake- Viskosimeter, Typ "Haake-Viscotester VT 2 plus, Hersteller: THERMO SCIENTIFIC FISHER, Braunschweig, Deutschland bei T = 20 °C mit 12 500 mPas gemessen.

Die Beschichtung des elastischen Nonwovens Typ 752 erfolgt in der Weise, dass die 10 cm breite Bahn mit der Transportgeschwindigkeit von 5 m/min einem Walzenrakelaggregat zugeführt wird, wobei möglichst geringe Zugkräfte verwendet werden, um den ungedehnten Zustand der Bahn Typ 752 weitgehend zu erhalten. Die Breite des Walzenrakels beträgt 15 cm. Die Warenbahn wird über die Walze und unter dem Rakelmesser durchgeführt und die pastöse Klebemasse vor dem Rakelmesser portionsweise aufgegeben, so dass immer eine Menge von ca. 100 g Klebemasse auf der Warenbahn aufliegt. Der Spalt zwischen Rakelmesser und Walze wird so eingestellt, dass der Nassauftrag des Klebers zwischen 55 g/m² und 65 g/m² gedehnt (nach DIN 61632 bei 3 N/cm) beträgt. Direkt hinter dem Walzenrakel durchläuft die mit Kleber beschichtete Warenbahn einen Trockenkanal, Länge 3,0 m, in welchem das Lösemittel (Isohexan) aus dem Kleber mit einem Warmluftstrom bei T = 80 °C verdampft wird. Der feste Klebstoff bildet einen mikroporösen, offenporigen Film von Kautschuk-Fragmenten. Die Bahnenware verlässt den Trockner im getrockneten Zustand und wird spannungsarm zu einer Rolle aufgewickelt. Die Bahnenware ist nun einseitig mit der trockenen Klebemasse beschichtet, die Auftragsmenge beträgt zwischen 7,2 g/m² und 8,5 g/m² Trockengewicht. Das Flächengewicht der einseitig beschichteten Bahnenware liegt bei 50,2 g/m² bis 51,5 g/m², die Dehnbarkeit bei ca. 190 % (beide Parameter nach DIN 61632 bei 3 N/cm). Die Rolle der einseitig beschichteten Bahnenware durchläuft nun in einem zweiten Durchlauf das Walzenrakel- Aggregat und den Trockenkanal wie oben beschrieben. Dabei wird die bisher unbeschichtete Seite des Substrats in analoger Arbeitsweise mit der Klebemasse beschichtet. Die nun beidseitig beschichtete Bahnenware verlässt den Trockner im getrockneten Zustand und wird spannungsarm zu einer Rolle aufgewickelt. Der feste Klebstoff bildet einen mikroporösen, offenporigen Film von Kautschuk-Fragmenten auf beiden Oberflächen des Substrats. Um zu vermeiden, dass die Lagen der Bahnen durch die haftende Kautschukoberfläche miteinander verkleben, wird beim Wickeln der Rolle ein Trennpapier oder eine Trennfolie als Abstandshalter zugeführt und zwischen die Lagen eingewickelt. Die Auftragsmenge des Klebers auf dem Substrat beträgt nun insgesamt zwischen 14,4 g/m² und 17,0 g/m² Trockengewicht. Das Flächengewicht der beidseitig beschichteten Bahnenware liegt bei 57,4 g/m² bis 60,0 g/m², die Dehnbarkeit bei ca. 160 % (beide Parameter nach DIN 61632 bei 3 N/cm).

Unter Verwendung eines Wickelkerns, z.B. Kunststoffhülse aus Polypropylen, Innendurchmesser 28 mm, Wandstärke 1 mm, Höhe 10,0 cm wird anschließend eine Länge von 1,75 m Bahnenware von der Rolle in spannungsarmem Zustand zu einem zylindrischen Wickelkörper aufgespult, welcher Ausgangsmaterial zur Herstellung der erfindungsgemäßen Fluidbinde ist. Das Nettogewicht (Bahnenware ohne Hülse) beträgt 27,0 g. Der Bindendurchmesser beträgt 54 mm.

Als Kühlfluid werden Mischungen Ethanol/ Wasser mit oder ohne Zusatz von Kühlhilfsmitteln verwendet:
Kühlfluid KF1: Wird durch Mischen folgender Komponenten hergestellt:
   48 g Ethanol- Wasser- Gemisch (mit 96 Vol.-% Ethanol) 50 g Wasser demineralisiert
   2,0 g Eumulgin HRE 40 (Emulgator, Hersteller COGNIS, Düsseldorf, Deutschland)
Kühlfluid KF2 (mit verstärkter Kühlwirkung): Mischen/Lösen folgender Komponenten
   45 g Ethanol- Wasser- Gemisch (mit 96 Vol.-% Ethanol)
   50 g Wasser demineralisiert
   0,05 g Kampfer
   0,05 g Menthol
   0,20 g Menthyllactat
   4,70 g Eumulgin HRE 40 (Emulgator, Hersteller COGNIS, Düsseldorf, Deutschland)

Zur Herstellung einer ersten Kühlbinde Typ KBF1 wird ein Wickelkörper in eine Kunststoffdose mit Schraubdeckel , Innendurchmesser 67 mm, Höhe 110 mm, gesetzt und mit 40,0 g Kühlfluid KF1 langsam übergossen, bis das Fluid vollständig von der Binde aufgesaugt ist. Das Fluid verteilt sich innerhalb einer Minute homogen in der Binde. Zur Herstellung einer zweiten Kühlbinde Typ KBF2 wird ein Wickelkörper in eine Kunststoffdose mit Schraubdeckel, Innendurchmesser 67 mm, Höhe 110 mm, gesetzt und mit 40,0 g Kühlfluid KF2 langsam übergossen, bis das Fluid vollständig von der Binde aufgesaugt ist. Das Fluid verteilt sich innerhalb einer Minute homogen in der Binde.

Nach 24 Stunden Wartezeit wurden die Binden einer physikalischen Prüfung unterzogen und folgende Ergebnisse ermittelt:

| Prüfergebnisse der Kühlbinden | Typ KBF1 | Typ KBF2 |
|---|---|---|
| Gewicht Fluidbinde (ohne Hülse) | 67,0 g | 67,0 g |
| Dehnbarkeit (DIN 61632 mit 3 N/cm) | 152 % | 158 % |
| Abrollkraft (cN/cm) | 35,8 cN/cm | 47,6 cN/cm |
| Haftkraft Seite A/B | 22,5 cN/cm | 20,8 cN/cm |
| AKTmax = max.Kühltemperaturdifferenz | - 4,2 K | - 5,5 K |

Die Kühltemperaturdifferenz ΔKT wird in einem Probanden-Anlegetest wie folgt ermittelt: Die Kühlbinde wird am Unterarm des Probanden in 5 - 10 cm Abstand vom Ellenbogen als 2- lagiger kantengerade überlappender Ring angewickelt und leicht angepresst, um die Lagen zu verbinden. Dann wird sofort ein Digitalthermometer mit Metallsensorspitze unter den Verband geschoben, so dass die Spitze mittig platziert ist. In gleicher Weise wurde zuvor die Ausgangstemperatur der Haut T(H) mit einer trockenen Idealbinde gemäß DIN 61632 ermittelt. Die Hauttemperatur unter der Kühlbinde T(B) wird in Intervallen von 1 Minute (anfangs) bis 15 Minuten von t = 0 bis max t = 180 Minuten Dauer gemessen. Die Kühltemperaturdifferenz ΔKT wird als Differenz T(B) - T(H) errechnet. ΔKTmax ist die maximale Kühltemperaturdifferenz, d.h. der Betrag T(B) - T(H) ist maximal.

Zur Anwendung werden die Binden am Unterschenkel unter leichter Dehnung von ca. 80 % mit 50 % Überlappung als Spiraltourenverband angewickelt und die Lagen von Hand gut angedrückt. Überraschenderweise gehen die Lagen eine haftende Verbindung ein, obwohl das Kühlfluid auch die Kautschukfragmente der Kohäsivbeschichtung bedeckt. Der Proband empfindet die kühlende Wirkung sofort nach Anlegen der Verbände, wobei der Höhepunkt der Kühlwirkung nach 30 - 60 Minuten erreicht wird. Die Kühlwirkung hält insgesamt ca. 90 bis 120 Minuten an. Subjektiv empfinden die Probanden bei der Binde KBF 2 eine höhere Kühlwirkung als bei der Binde KBF1.

Darüber hinaus betrifft die Erfindung auch ein Verfahren zur Herstellung einer Bandage umfassend eine Flachmaterialbahn als Substrat, wobei auf beide Flachseiten des Substrats eine selbsthaftende kohäsive Klebemasse zumindest partiell aufgebracht ist, wobei das Aufbringen der Klebemasse in Form eines wasserfreien kohäsiven Haftklebstoffs umfassend Natur- und/oder Synthesekautschuk erfolgt, bei dem der Natur- und/oder Synthesekautschuk zum Aufbringen in organischem Lösungsmittel gelöst oder zum Aufbringen schmelzflüssig ist und danach entweder das Lösungsmittel verdampft oder der Haftklebstoff aushärtet und danach das mit der kohäsiven Klebemasse beschichtete Substrat mit einer flüssigen Zubereitung getränkt wird.

### Beispiel 2 : Kohäsive Webbinde mit essigsaurer Tonerde :

Als bahnförmiges Substrat wird eine elastische Gewebekonstruktion Typ 199, Hersteller: Karl Otto Braun GmbH & Co. KG, Wolfstein, Deutschland, verwendet, wobei die Elastizität des Grundtextils in Kettrichtung aus der Kombination von dauerelastischen Elastanfäden mit starren Baumwollgarnen resultiert. Einzelheiten dieses Trägertextils siehe nachstehende Tabelle:

| **Materialaufbau Gewebe Typ 199** | 99 % Baumwolle, 1 % Elastan |
|---|---|
| Kettfaden - Material | Kettfaden A : 20 tex Baumwolle |
| | Kettfaden β : 15,6 tex Lycra (Elastan) umflort mit 24 tex Baumwolle |
| Kettfolge | 4 Fäden A - 1 Faden B |
| Fadenzahl / Kettdichte | 110 pro 10 cm Breite |
| Schussfaden - Material | 50 tex Baumwolle |
| Fadenzahl / Schussdichte | 130 pro 10 cm gedehnt (DIN 61632) |
| Flächenqewicht gedehnt | 95 g/ qm |
| Elastizität | In Längsrichtung (Kettrichtung) |
| Dehnbarkeit / Rückzug nach DIN 61632 | 120 % / 99 % |

Die Beschichtung des elastischen Gewebes Typ 199 erfolgt unter Verwendung der im Beispiel 1 beschriebenen Klebemasse auf der gleichen Walzenrakel- Beschichtungsanlage, unter Verwendung einer 10 cm breiten Bahn. Die Auftragsmenge des Klebers auf dem Substrat beträgt nun insgesamt 13,0 g Trockengewicht. Das Flächengewicht der beidseitig beschichteten Bahnenware liegt bei 108 g , die Dehnbarkeit bei 100 % (beide Parameter nach DIN 61632 bei 3 N/cm).

Unter Verwendung eines Wickelkerns, z.B. Kunststoffhülse aus Polypropylen, Innendurchmesser 28 mm, Wandstärke 1 mm, Höhe 10,0 cm wird eine Länge von 2,0 m Bahnenware des Gewebes Typ 199 in spannungsarmem Zustand zu einem zylindrischen Wickelkörper aufgespult, welcher Ausgangsmaterial zur Herstellung der erfindungsgemäßen fluidimprägnierten Bandage ist.

Das Nettogewicht (Bahnenware ohne Hülse) beträgt 44,0 g. Der Bindendurchmesser 57 mm.

Als Fluid wird eine verdünnte wässrige Lösung von Aluminiumacetat-tartrat ("essigsaure Tonerde" standardisiert siehe Deutsches Arzneimittelbuch, Ausgabe 2012 (DAB 2012)) verwendet. Dieser Wirkstoff hat eine antiseptische, adstringierende und schwellungshemmende Wirkung und wird z.B. bei Kontusionen verwendet.

Fluid KF3: Wird durch Mischen folgender Komponenten hergestellt:
10 g Aluminiumacetat- tartrat - Lösung nach DAB 2012 90 g Wasser demineralisiert
2,0 g Eumulgin HRE 40 (Emulgator, Hersteller COGNIS, Düsseldorf, Deutschland)

Zur Herstellung der Fluidbinde (KBF3) gemäß Beispiel 2 wird ein Wickelkörper in eine Kunststoffdose mit Schraubdeckel , Innendurchmesser 67 mm, Höhe 110 mm, gesetzt und mit 44,0 g Fluid KF3 langsam übergossen, bis das Fluid vollständig von der Binde aufgesaugt ist. Das Fluid verteilt sich innerhalb einer Minute homogen in der Binde.

Nach 24 Stunden Wartezeit wurden die Binden einer physikalischen Prüfung unterzogen und folgende Ergebnisse ermittelt:

| Prüfergebnisse der Kühlbinden | Typ KBF3 |
|---|---|
| Gewicht Fluidbinde (ohne Hülse) | 88,0 g |
| Dehnbarkeit (DIN 61632 mit 3 N/cm) | 99 % |
| Abrollkraft (cN/cm) | 72,7 cN/cm |
| Haftkraft Seite A/B | 28,5 cN/cm |

Zur Anwendung werden die Binden am Unterschenkel unter leichter Dehnung von ca. 60 % mit 50 % Überlappung als Spiraltouren- Verband angewickelt und die Lagen von Hand gut angedrückt. Überraschenderweise gehen die Lagen eine haftende Verbindung ein, obwohl das Fluid aus essigsaurer Tonerde- Lösung eine Grenzschicht zwischen den Kautschukfragmenten der Kohäsivbeschichtung bildet. Der Proband empfindet die Wirkung als angenehm kühlend und hauttrocknend (adstringierend). Schwellungen bilden sich zurück.

## Patentansprüche

1. Bandage umfassend ein Flachbahnmaterial als Substrat, wobei auf beide Flachseiten des Substrats eine selbsthaftende kohäsive Klebemasse zumindest partiell aufgebracht ist und das Substrat mit einer flüssigen Zubereitung getränkt ist, **dadurch gekennzeichnet, dass** die Klebemasse einen wasserfreien kohäsiven Haftklebstoff umfassend Natur- und/oder Synthesekautschuk umfasst, bei dem der Natur- und/oder Synthesekautschuk zum Aufbringen in organischem Lösungsmittel gelöst ist oder der Haftklebstoff zum Aufbringen schmelzflüssig ist.

2. Bandage nach Anspruch 1, **dadurch gekennzeichnet, dass** der Haftkleber aus Natur- und/oder Synthesekautschuk besteht.

3. Bandage nach Anspruch 1 oder 2, **dadurch gekennzeichnet, dass** die Bandage in Längs- und/oder Querrichtung elastisch ist.

4. Bandage nach einem der Ansprüche 1 bis 3, **dadurch gekennzeichnet, dass** die flüssige Zubereitung eine einphasige Lösung, mehrphasige Emulsion, ein Gel oder ein Schaum ist.

5. Bandage nach Anspruch 4, **dadurch gekennzeichnet, dass** die flüssige Zubereitung eine ein- oder mehrphasige Zubereitung auf Basis von Wasser mit Zusätzen von anorganischen Stoffen, insbesondere Salzen oder organischen Stoffen, insbesondere Alkoholen, Estern, Ethern, Ketonen oder Terpenen ist.

6. Bandage nach einem der vorangehenden Ansprüche,
**dadurch gekennzeichnet, dass** das Substrat ein Gewebe, Gewirke, Gestrick oder Vlies ist.

7. Bandage nach einem der vorangehenden Ansprüche,
**dadurch gekennzeichnet, dass** das Substrat elastische Fäden, insbesondere hochgedrehte Baumwollfäden als Spinn- oder Zwirnkreppfäden, texturierten Polyamid- und/oder Polyestergarne, Gummi- und/oder Polyurethan-Elastanfäden oder Kombinationen hiervon umfasst.

8. Bandage nach einem der vorangehenden Ansprüche,
**dadurch gekennzeichnet, dass** die kohäsive Beschichtung offenporig ausgebildet ist.

9. Bandage nach einem der vorangehenden Ansprüche,
**dadurch gekennzeichnet, dass** die Klebemasse in Mustern aufgebracht ist.

10. Bandage nach einem der vorangehenden Ansprüche,
**dadurch gekennzeichnet, dass** der Auftrag der Klebemasse 2 bis 20 g/m² beträgt.

11. Verfahren zum Herstellen einer Bandage nach einem der vorangehenden Ansprüche, **dadurch gekennzeichnet, dass** ein vorzugsweise elastisches Substrat in Form von einem Flachbahnmaterial beidseitig zumindest partiell mit einem kohäsiv selbsthaftenden, wasserfreien Haftklebstoff umfassend Natur- und/oder Synthesekautschuk, bei dem der Natur- und/oder Synthesekautschuk zum Aufbringen in organischem Lösungsmittel gelöst ist oder der Haftkleber beim Aufbringen schmelzflüssig ist, beschichtet wird und das Flachbahnmaterial danach zu Binden konfektioniert und die Binde dann mit einer flüssigen Zubereitung imprägniert wird.

12. Verfahren nach Anspruch 11, **dadurch gekennzeichnet,**
**dass** der kohäsive Haftklebstoff zuerst auf einer und nachfolgend der zweiten Flachseite des Flachbahnmaterials aufgebracht wird.

## Claims

1. A bandage comprising a flat-web material as substrate, wherein a self-adhesive cohesive adhesive has been at least partially applied on both flat sides of the substrate and the substrate has been impregnated with a liquid preparation, **characterized in that** the adhesive comprises an anhydrous cohesive pressure-sensitive adhesive comprising natural rubber and/or synthetic rubber, in which the natural rubber and/or synthetic rubber has been dissolved in an organic solvent for the purposes of application or the pressure-sensitive adhesive is a melted liquid for the purposes of application.

2. The bandage as claimed in claim 1, **characterized in that** the pressure-sensitive adhesive consists of natural rubber and/or synthetic rubber.

3. The bandage as claimed in claim 1 or 2, **characterized in that** the bandage is elastic in the longitudinal direction and/or transverse direction.

4. The bandage as claimed in any of claims 1 to 3, **characterized in that** the liquid preparation is a single-phase solution, multiphase emulsion, a gel or a foam.

5. The bandage as claimed in claim 4, **characterized in that** the liquid preparation is a single-phase or multiphase preparation based on water with additions of inorganic substances, more particularly salts, or organic substances, more particularly alcohols, esters, ethers, ketones or terpenes.

6. The bandage as claimed in any of the preceding claims, **characterized in that** the substrate is a woven fabric, warp-knitted fabric, weft-knitted fabric or nonwoven.

7. The bandage as claimed in any of the preceding claims, **characterized in that** the substrate comprises elastic threads, more particularly high-twist cotton threads as spun or twisted crepe threads, textured polyamide and/or polyester yarns, rubber and/or polyurethane elastane threads or combinations thereof.

8. The bandage as claimed in any of the preceding claims, **characterized in that** the cohesive coating is openpore.

9. The bandage as claimed in any of the preceding claims, **characterized in that** the adhesive has been applied in patterns.

10. The bandage as claimed in any of the preceding claims, **characterized in that** the application of the adhesive is 2 to 20 g/m².

11. A method for producing a bandage as claimed in any of the preceding claims, **characterized in that** a preferably elastic substrate in the form of a flat-web material is at least partially coated on both sides with an anhydrous cohesive pressure-sensitive adhesive comprising natural rubber and/or synthetic rubber, in which the natural rubber and/or synthetic rubber has been dissolved in an organic solvent for the purposes of application or the pressure-sensitive adhesive is a melted liquid for the purposes of application the flat-web material is then converted to bandages and the bandage is then impregnated with a liquid preparation.

12. The method as claimed in claim 10, **characterized in that** the cohesive adhesive is applied first to one flat side of the flat-web material and then to the second flat side of said flat-web material.

## Revendications

1. Bandage comprenant un matériau de bande plat en tant que substrat, dans lequel une masse adhésive cohésive auto-adhésive est appliquée au moins partiellement sur les deux faces plates du substrat et le substrat est imprégné d'une préparation liquide, **caractérisé par le fait que** ladite masse adhésive comprend une colle de contact cohésive anhydre comprenant du caoutchouc naturel et/ou synthétique, dans lequel le caoutchouc naturel et/ou synthétique est dissous dans un solvant organique pour l'application ou la colle de contact est fondue pour l'application.

2. Bandage selon la revendication 1, **caractérisé par le fait que** la colle de contact est constituée de caoutchouc naturel et/ou synthétique.

3. Bandage selon la revendication 1 ou 2, **caractérisé par le fait que** le bandage est élastique dans le sens longitudinal et/ou dans le sens transversal.

4. Bandage selon l'une quelconque des revendications 1 à 3, **caractérisé par le fait que** la préparation liquide est une solution monophasique, une émulsion multiphasique, un gel ou une mousse.

5. Bandage selon la revendication 4, **caractérisé par le fait que** la préparation liquide est une préparation monophasique ou multiphasique à base d'eau additionnée de substances inorganiques, en particulier de sels ou de substances organiques, en particulier d'alcools, d'esters, d'éthers, de cétones ou de terpènes.

6. Bandage selon l'une quelconque des revendications précédentes, **caractérisé par le fait que** le substrat est un tissu, un tissu à mailles, un tricot ou un nontissé.

7. Bandage selon l'une quelconque des revendications précédentes, **caractérisé par le fait que** le substrat comprend des fils élastiques, en particulier des fils de coton retordus comme fils crêpe de filage ou fils crêpe retors, des fils texturés de polyamide ou de polyester, des fils de caoutchouc ou de polyuréthane élasthanne ou des combinaisons de ceux-ci.

8. Bandage selon l'une quelconque des revendications précédentes, **caractérisé par le fait que** le revêtement cohésif est réalisé à pores ouverts.

9. Bandage selon l'une quelconque des revendications précédentes, **caractérisé par le fait que** la masse adhésive est appliquée en motifs.

10. Bandage selon l'une quelconque des revendications précédentes, **caractérisé par le fait que** l'application de la masse adhésive est de 2 à 20g/m².

11. Procédé de fabrication d'un bandage selon l'une quelconque des revendications précédentes, **caractérisé par le fait qu'**un substrat de préférence élastique sous la forme d'un matériau de bande plat est au moins partiellement recouvert sur les deux faces d'une colle de contact cohésive auto-adhésive anhydre comprenant du caoutchouc naturel et/ou synthétique, dans lequel le caoutchouc naturel et/ou synthétique est dissous dans un solvant organique pour l'application ou la colle de contact est fondue lorsqu'elle est appliquée, et le matériau de bande plat est ensuite confectionné pour former des bandes et, puis, la bande est imprégnée d'une préparation liquide.

12. Procédé selon la revendication 11, **caractérisé par le fait que** la colle de contact cohésive est appliquée d'abord sur une face plate et ensuite sur la deuxième face plate du matériau de bande plat.
